**Europäisches Patentamt**

**European Patent Office**

**Office européen des brevets**

(19)

(11) Veröffentlichungsnummer: **0 028 036**
**B1**

(12) # EUROPÄISCHE PATENTSCHRIFT

(45) Veröffentlichungstag der Patentschrift:
13.08.86

(21) Anmeldenummer: 80200882.1

(22) Anmeldetag: 18.09.80

(51) Int. Cl.⁴: **A 61 B 6/03**

(54) Verfahren und Anordnung zur Untersuchung eines Körpers mit durchdringender Strahlung.

(30) Priorität: 27.09.79 DE 2939146

(43) Veröffentlichungstag der Anmeldung:
06.05.81 Patentblatt 81/18

(45) Bekanntmachung des Hinweises auf die Patenterteilung:
13.08.86 Patentblatt 86/33

(84) Benannte Vertragsstaaten:
DE FR GB IT NL SE

(56) Entgegenhaltungen:
DE-A-2 720 759
DE-A-2 828 240
DE-B-2 604 020
FR-A-1 561 351
FR-A-2 386 055
FR-A-2 441 855

(73) Patentinhaber: **Philips Patentverwaltung GmbH, Billstrasse 80, D-2000 Hamburg 28 (DE)**
(84) Benannte Vertragsstaaten: **DE**

(73) Patentinhaber: **N.V. Philips' Gloeilampenfabrieken, Groenewoudseweg 1, NL- 5621 BA Eindhoven (NL)**
(84) Benannte Vertragsstaaten: **FR GB IT NL SE**

(72) Erfinder: **Harding, Geoffrey, Dr., Kastanienallee 22, D-2084 Rellingen (DE)**
Erfinder: **Wagner, Wolfgang, Bookholtstwiete 11, D-2000 Hamburg (DE)**

(74) Vertreter: **Hartmann, Heinrich, Dipl.- Ing., Philips Patentverwaltung GmbH Billstrasse 80 Postfach 10 51 49, D-2000 Hamburg 28 (DE)**

EP 0 028 036 B1

### Beschreibung

Die Erfindung betrifft ein Verfahren zur Korrektur der mit Hilfe einer aus mehreren Detektorelementen bestehenden Detektoranordnung bei der Durchstrahlung eines Körpers mit durchdringender Strahlung gemessen werde, bei dem ein Primärstrahl mit im Vergleich zu diesem geringen Querschnitt den Körper durchsetzt und die dabei erzeugte Streustrahlung durch eine Blendenanordnung ausgeblendet und durch die Detektoranordnung gemessen wird, wonach aus den Meßwerten die Dichteverteilung im Primärstrahl durchsetzten Bereich des Körpers rekonstruiert wird, sowie eine Anordnung zur Durchführung des Verfahrens. Ein solches Verfahren ist aus der DE-OS 27 13 581 bekannt. Die Blendenanordnung besitzt dabei eine schlitzförmige Blendenöffnung, die etwa senkrecht zum Zentralstrahl verläuft. Durch diese schlitzförmige Blendenöffnung ergibt sich eine eindeutige Zuordnung zwischen einem Punkt auf dem Primärstrahl und einen Detektorelement innerhalb der Detektoranordnung. Jedes Detektorelement mißt dabei die Streustrahlung aus einem anderen Teilbereich des Primärstrahls, die ein Maß für die Dichte in diesem Teilbereich ist.

Obwohl hierbei also Streustrahlung zur Ermittlung der Dichteverteilung ausgenutzt wird, werden die mit der Detektoranordnung ermittelten Meßergebnisse durch Streustrahlung verfälscht. Ein Teil der in den Körper im Bereich des Primärstrahls erzeugten Streustrahlung wird in dem Körper nämlich noch ein weiteres Mal oder gar mehrmals gestreut und trifft durch die schlitzförmige Blendenöffnung in der Regel auf einen anderen Detektor als die Streustrahlung, die vom Ursprungspunkt der mehrfach gestreuten Streustrahlung (im folgenden kurz als Mehrfach-Streustrahlung bezeichnet - im Gegensatz zu der im Primärstrahl erzeugten Einfach-Streustrahlung) aus direkt auf die Detektoranordnung trifft.

Zur Unterdrückung dieser Mehrfach-Streustrahlung ist es bereits bekannt, vor der Detektoranordnung Lamellen anzuordnen, die aus die Strahlung stark absorbierenden Material (Blei o.dgl.) bestehen und die sich in Ebenen befinden, die sich ungefähr im Primärstrahl schneiden. Damit kann man zwar die außerhalb der erwähnten Ebenen verlaufende Streustrahlung weitgehend unterdrücken, jedoch nicht die in diesen Ebenen werlaufende Mehrfach-Streustrahlung.

Aufgabe der worliegenden Erfindung ist es daher, den Einfluß der Streustrahlung durch deren Messung und entsprechende Korrektur der Dichteverteilung weiter zu werringern.

Ausgehend won einem Verfahren der eingangs genannten Art wird diese Aufgabe dadurch gelöst, daß wenigstens ein Teil der Elemente der Detektoranordnung zumindest zeitweise gegen die Einfach-Streustrahlung abgeschirmt wird und daß die dabei von den abgeschirmten Elementen gemessenen Werte der Mehrfach-Streustrahlung zur Korrektur der bei der Messung der Einfach-Streustrahlung erzeugten Meßwerte herangezogen werden.

Dabei wird won der Überlegung ausgegangen, daß die Meßwerte aus der additiwen Überlagerung eines durch die Einfach-Streustrahlung hervorgerufenen und eines durch die Mehrfach-Streustrahlung hervorgerufenen Anteils entstehen, wobei die örtliche Verteilung der Intensitäten des durch Mehrfach-Streustrahlung hervorgerufenen Anteils geringeren Schwankungen unterliegt als die der Intensitäten der Einfach-Streustrahlung. Wenn nun wenigstens ein Teil der Detektorelemente zumindest zeitweise gegen die Einfach-Streustrahlung abgeschirmt wird, erfaßt dieser Teil der Detektorelemente nur die Mehrfach-Streustrahlung und die von diesen Detektorelementen gemessenen (Korrektur-) Werte werden dann von den (durch Messung der Einfach-Streustrahlung und der unerwünschten Mehrfach-Streustrahlung erhaltenen) Meßwerten abgezogen.

Eine Ausführungsform zur Durchführung des erfindungsgemäßen Verfahrens, die ausgeht von einer Anordnung mit wenigstens einer Strahlenquelle zur Erzeugung des Primärstrahles, mit einer Detektoranordnung zur Messung der im Primärstrahl erzeugten Streustrahlung mit einer zwischen der Detektoranordnung und dem Primärstrahl angeordneten Blendenanordnung mit schlitzförmiger mit ihrer Längsrichtung senkrecht zum Primärstrahl verlaufender Blendenöffnung, und mit einer Vielzahl zwischen der Blendenöffnung und der Detektoranordnung angeordneter Lamellen aus die Strahlung absorbierenden Material, die in sich im Körper schneidenden Ebenen liegen, ist gekennzeichnet durch Mittel zur Erzeugung einer Relativbewegung zwischen dem Primärstrahl einerseits und der Blendenanordnung der Detektoranordnung und den Lamellen andererseits derart, daß die Lamellenebenen von einer Position, in der sie sich im Primärstrahl schneiden, in eine Position bevegbar sind, in der sie sich außerhalb des Primärstrahls schneiden.

Bei einer ersten Ausführungsform wird diese Relatiwbewegung erreicht durch eine Antriebseinrichtung zur Verschiebung der Detektoranordnung mitsamt der Blendenanordnung und den Lamellen in Längsrichtung der schlitzförmigen Blendenöffnungen. Ist dabei die Detektoranordnung mitsamt den Blenden und den Lamellen genügend weit zur Seite verschoben, dann kann nur noch Mehrfach-Streustrahlung von den Detektoren gemessen werden. Die auf diese Weise ermittelten (Korrektur-) Werte müssen anschließend mit einem Faktor multipliziert werden, der die durch die obengenannte Verschiebung verursachte Änderung der mittleren Mehrfach-Streuintensitäten rückgängig macht, bevor sie zur Korrektur der Meßwerte von diesen abgezogen werden. Dieser Faktor kann einmal aus Messungen an einem Phantom berechnet werden.

Bei einer zweiten Ausführungsform wird die Relativbewegung zwischen den Primärstrahlen und der Blendenanordnung der Detektoranordnung und der Lamelle dadurch erreicht, daß vor der Strahlenquelle eine feststehende Blende angeordnet ist mit wenigstens zwei Öffnungen zum Ausblenden der Primärstrahlung, daß vor dieser Blende eine mit einer Antriebseinrichtung verbundene drehbare Absorberscheibe angeordnet ist, die mit bezüglich der Drehachse kreisbogenförmigen Ausnehmungen versehen ist, die gegeneinander auf dem Umfang versetzt und in solchem Abstand von der Drehachse angeordnet sind, daß jeweils höchstens einer der durch die Blende ausgeblendeten Strahlen durch eine Ausnehmung in der Absorberscheibe treten kann.

In diesem Fall ändert also der Primärstrahl seine Lage in bezug auf die Blendenanordnung.

Nach einer Weiterbildung dieser zweiten Ausführungsform ist vorgesehen, daß die Ausnehmung, durch die derjenige Primärstrahl hindurchtritt, in dem sich die Lamellenebenen schneiden, eine größere Bogenlänge hat als die andere(n) Ausnehmung(en). Dadurch wird die Belastung des Patienten bei der Erfassung der Mehrfach-Streustrahlung herabgesetzt.

Bei der bisher beschriebenen Ausführungsform waren die Röntgenstrahlung absorberende sich im Primärstrahl schneidende Lamellen erforderlich. Bei der folgenden Ausführungsform der Erfindung kann auf Lamellen verzichtet werden, so daß der Aufbau der Detektoranordnung wereinfacht und seine Empfindlichkeit erhöht wird. Dabei ist vorgesehen, daß in die Blendenöffnung enthaltenden Ebenen jeweils wenigstens zwei Detektoren zur Lieferung zweier getrennter Signale vorgesehen sind, von denen einer durch ein Absorberstück gegen Einfach-Streustrahlung abgeschirmt ist. Bei dieser Ausführungsform werden also die Meßwerte (die durch Einfach- und Mehrfach-Streustrahlung hervorgerufen werden) won dem bzw. den nicht abgeschirmten Detektor(en) und die Mehrfach-Streustrahlung won dem gegen Einfach-Streustrahlung abgeschirmten Detektorelement gleichzeitig gemessen.

Die Erfindung wird nachstehend anhand der Zeichnung näher erläutert. Es zeigen

Fig. 1 eine perspektivische Darstellung eines Gerätes,

Fig. 2 eine schematische Darstellung des Gerätes in einer zum Primärstrahl senkrechten Ebene,

Fig. 3 die Anordnung nach einer Verschiebung der Blendenanordnung,

Fig. 4 eine Anordnung zur Verarbeitung der bei dem erfindungsgemäßen Verfahren entstehenden Signale,

Fig. 5 eine Einrichtung zur Verschiebung des Primärstrahles in bezug auf die Blende in einer zum Primärstrahl senkrechten Ebene,

Fig. 6 dieselbe Einrichtung in einer den Primärstrahl enthaltenden Ebene, und

Fig. 7 eine weitere Ausführungsform.

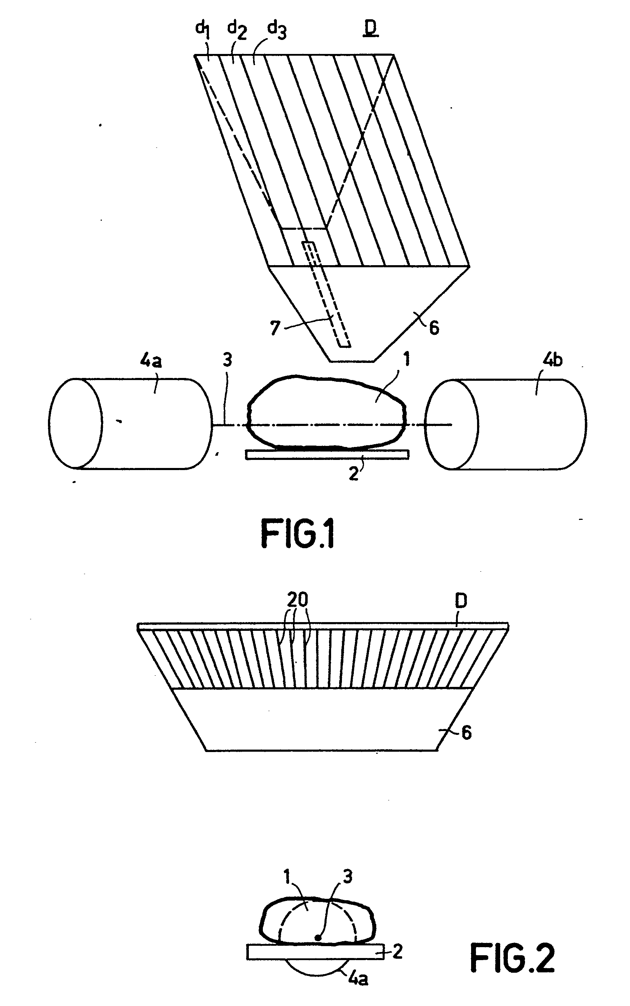

In Fig. 1 ist der zu untersuchende Körper 1 auf einer Tischplatte 2 liegend dargestellt. Dieser Körper wird won einem Primärstrahl 3 durchsetzt, der von zwei beiderseits des Körpers angeordneten Röntgenstrahlern 4a und 4b erzeugt und durch eine nicht näher dargestellte Ausblendvorrichtung ausgeblendet wird. Die Abmessungen des ausgeblendeten Primärstrahles bestimmen das Auflösungsvermögen der Anordnung. Je kleiner sein Querschnitt ist, um so besser ist das Auflösungsvermögen.

Die bei der Untersuchung an die in den Röntgenstrahlern 4a bzw. 4b enthaltenen Röntgenröhren angelegte Spannung beträgt ungefähr 350 kV. Dadurch wird einerseits die Strahlenbelastung für den Patienten klein gehalten und andererseits ist dabei die Schwächung des Primärstrahles durch Photoabsorption klein im Vergleich zur Schwächung durch Compton-Streuung.

Die in dem vom Primärstrahl 3 durchsetzten Bereich des Körpers erzeugte Streustrahlung erreicht durch die schlitzförmige vorzugsweise verstellbare Öffnung 7 einer oberhalb des zu untersuchenden Körpers angeordneten Blendenanordnung 6 eine Detektoranordnung D, die sich aus einer größeren Anzahl von Detektorelementen $d_1$, $d_2$, $d_3$ usw. zusammensetzt, die nebeneinander auf einer zum Primärstrahl parallelen Geraden angeordnet sind. Wie Fig. 1 zeigt, hat die als Meßfläche wirksame Oberfläche der Detektoren die Form eines langgestreckten Rechteckes, dessen Längsseiten in einer zum Primärstrahl senkrechten Ebene liegen. Die schlitzförmige Öffnung 7 der Blendenanordnung 6 hat eine entsprechende Form, wobei allerdings ihre Abmessungen in beiden Richtungen im gleichen Verhältnis kleiner sind wie ihr Abstand vom Primärstrahl 3 kleiner ist als derjenige der Detektorelemente.

Durch die schlitzförmige Öffnung 7 in der Blendenanordnung 6 ergibt sich eine eindeutige Zuordnung zwischen einem Punkt auf dem Primärstrahl 3 und einem Detektorelement innerhalb der Detektorgruppe D. Die won dem Primärstrahl in einem bestimmten Punkt erzeugte Streustrahlungskeule trifft die Detektoranordnung D längs eines bestimmten Streifens, der einem Detektorelement, gegebenenfalls auch zwei benachbarten Detektorelementen, zugeordnet ist. Auf diese Weise "sieht" also jedes Detektorelement die von einem bestimmten Punkt bzw. Bereich des Primärstrahls ausgehende Streustrahlung, wobei jeder Punkt bzw. Bereich auf dem Primärstrahl einem anderen Detektor zugeordnet ist.

Durch eine Relativverschiebung zwischen dem Körper 1 einerseits und dem ihn durchsetzenden Primärstrahl 3 andererseits kann anschließend ein anderer Bereich des Körpers durchstrahlt und die Dichteverteilung mittels der Detektoranordnung D erfaßt werden. Wiederholt man diesen Vorgang für eine Vielzahl von Positionen, dann kann auf diese Weise die Dichteverteilung auf einer beliebigen Fläche des Körpers erfaßt werden, die nicht notwendigerweise eine Ebene sein muß.

Unterhalb der Tischplatte 2 kann noch eine identisch aufgebaute Blenden- und Detektoranordnung angeordnet sein. Die Detektorelemente, die jeweils in derselben zum Primärstrahl 3 senkrechten Ebene liegen, erfassen die Streustrahlung desselben Bereiches im Primärstrahl; ihre Ausgangssignale können daher addiert werden.

Fig. 2 zeigt den senkrecht zur Zeichenebene durch den auf dem Tisch 2 gelagerten Körper 1 verlaufenden Querschnitt des Primärstrahls 3 sowie die oberhalb des Primärstrahls angeordnete Blendenanordnung 6 und die Detektoranordnung D. Die Längsrichtung der einzelnen Detektorelemente und des Schlitzes verläuft bei dieser Darstellung horizontal. Innerhalb der Blendenanordnung 6 ist vor der Detektoranordnung D eine Vielzahl von Lamellen 20 angeordnet. Die Lamellen liegen in zur Zeichenebene senkrecht verlaufenden Ebenen, die sich im Primärstrahl 3 schneiden. Die Lamellen werlaufen somit senkrecht zur Längsrichtung der einzelnen Detektorelemente und senkrecht zur Längsrichtung des Schlitzes. Die Einfach-Streustrahlung erreicht die Detektoranordnung D dabei praktisch ungeschwächt, während die Mehrfach-Streustrahlung durch die Bleilamellen 20 absorbiert wird, wenn sie nicht in den Primärstrahl enthaltenden Ebenen verläuft.

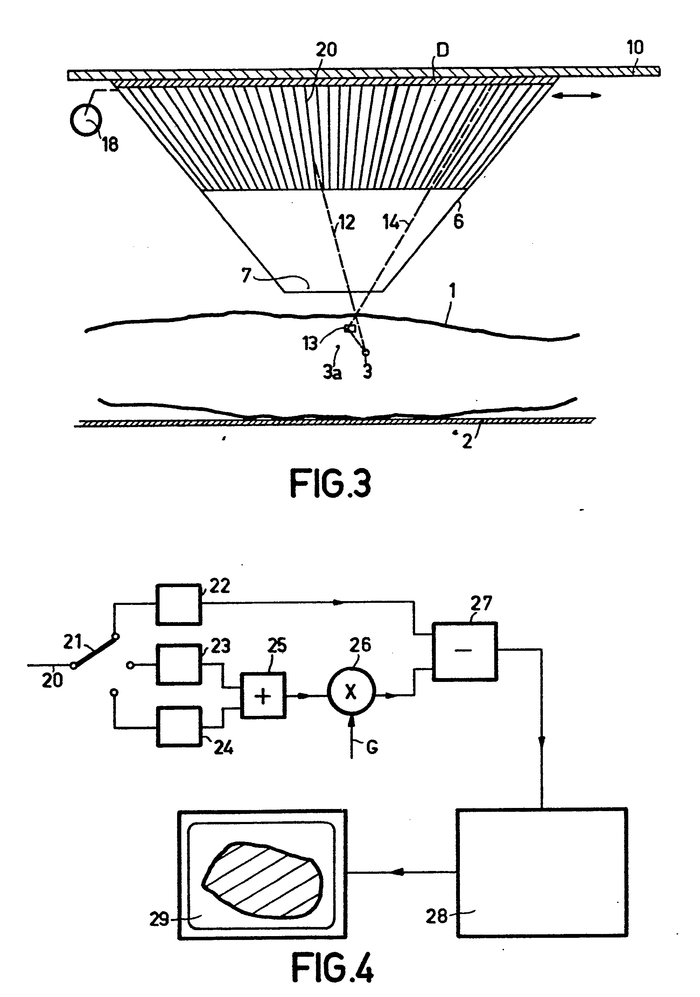

Fig. 3 zeigt, daß die Blendenanordnung 6 mitsamt der Detektoranordnung D und den Lamellen 20 an einer fest angeordneten Schiene 10 mittels eines Motorantriebes 18 verschiebbar ist. Die Schiene 10 verläuft parallel zur Längsrichtung der schlitzförmigen Öffnung 7 und damit senkrecht zum Primärstrahl 3. Die Blendenanordnung ist dabei seitlich verschoben worden, so daß sich die Lamellenebenen 20 nicht mehr im Primärstrahl 3 schneiden, sondern in der zur Zeichenebene senkrechten Achse 3a; der Abstand zwischen 3 und 3a gibt die Verschiebung gegenüber der Position in Fig. 2 an. Man erkennt, daß die vom Primärstrahl 3 ausgehende Einfach-Streustrahlung - in der Zeichnung ist nur beispielsweise der Strahl 12 gezeichnet - die Detektoranordnung D nicht erreichen kann, weil sie von einer der Bleilamellen 20 absorbiert wird. Hingegen kann Mehrfach-Streustrahlung - soweit sie in Richtung der Lamellenebenen verläuft - hier ist beispielhaft der am Element 13 zum zweitenmal gestreute Strahl 14 eingezeichnet - die Detektoranordnung D durch die Lamellen hindurch erreichen. Da die Intensität der Mehrfach-Streustrahlung sich räumlich relatiw wenig verändert, kann dawon ausgegangen werden, daß die in dieser Position von den Detektorelementen gemessene Mehrfach-Streustrahlung annähernd dem Mehrfach-Streustrahlenanteil entspricht, den die Detektorelemente (zusätzlich zur Einfach-Streustrahlung) messen, wenn in der Position der Fig. 2 die Lamellen auf den Primärstrahl 3 ausgerichtet sind. Es ist zweckmäßig, in einem dritten Schritt die Blendenanordnung zur anderen Seite zu verschieben, so daß sich die Lamellenebenen in einer Achse rechts vom Primärstrahl 3 schneiden und über die in dieser Position sowie in der in Fig. 3 dargestellten Position ermittelten Mehrfach-Streustrahlen Meßwerte zu ermitteln.

Fig. 4 zeigt eine Anordnung zur Verarbeitung der auf diese Weise erhaltenen Werte. Die von einem Detektorelement der Detektoranordnung gelieferten Meßwerte gelangen - gegebenenfalls nach Digitalisierung - über eine Leitung 20 und einen Multiplexer 21 zu einem won drei Zwischenspeichern 22, 23 oder 24. Der Multiplexer 21 wird von einer nicht näher dargestellten Steuereinrichtung so gesteuert, daß der erste Meßwert, der in der Stellung gemessen wird, in der sich die Lamellenebenen im Primärstrahl schnäiden, in den Speicher 22 gelangt, während die in den beiden anderen seitlich davon befindlichen Stellungen der Blendenanordnung gemessenen Meßwerte in die Zwischenspeicher 23 und 24 eingeschrieben werden. Die in den Zwischenspeichern 23 und 24 enthaltenen Werte werden mit einer Addiereinrichtung 25 summiert und in einer Multipliziereinrichtung 26 mit einem Faktor G multipliziert. Dieser Faktor ist so gewählt, daß die Unterschiede in den Intensitäten bei der Erfassung der Einfach-Streustrahlung (in der in Fig. 2 dargestellten Position) einerseits und der Mehrfach-Streustrahlung andererseits (bei der Erfassung der Mehrfach-Streustrahlung ist die Intensität des Primärstrahls zum Beispiel geringer) sowie gegebenenfalls auch in der Bestrahlungsdauer der Detektorelemente wieder rückgängig gemacht werden. Das Ausgangssignal der Multipliziereinrichtung 26 ist daher ein Maß für die Intensität der Mehrfach-Streustrahlung in der ersten Position (Fig. 2). Dieses Signal wird in der Subtrahiereinrichtung 27 von dem im Zwischenspeicher 22 gespeicherten Signal subtrahiert, das ein Maß für die gesamte Streustrahlung (Einfach-Streustrahlung + Mehrfach-Streustrahlung) darstellt. Am Ausgang der Subtrahierschaltung 27 ergibt sich daher ein Signal, das im wesentlichen nur die won dem betreffenden Detektorelement gemessene Einfach-Streustrahlung darstellt und daß der Rechen- und Speichereinrichtung 28 zugeführt wird.

Für die anderen Detektorelemente können ebenfalls den Elementen 20 bis 27 entsprechende Elemente vorgesehen sein. Es ist jedoch auch möglich, die Ausgangssignale mehrerer benachbarter Detektorelemente in den Stellungen, in denen die Lamellen sich außerhalb des Primärstrahles schneiden, zusammenzufassen, wobei sich eine räumliche Integration über die Mehrfach-Streustrahlung ergibt, und diese Werte von den in der Position gemäß Fig. 2 gemessenen, die gesamte Streustrahlung (Einfach- + Mehrfach-- Streustrahlung) repräsentierenden Meßwerten abzuziehen. Dabei muß selbstverständlich der Gewichtungsfaktor anders gewählt werden. Ebenso ist es aufgrund der Tatsache, daß sich die Mehrfach-Streustrahlung räumlich nur relativ wenig ändert, möglich, zur Erfassung der Mehrfach-Streustrahlung nur einen Teil der Detektorelemente, z.B. jedes vierte Detektorelement, heranzuziehen und dessen Ausgangssignale zur Korrektur der auch von den benachbarten Detektorelementen gelieferten Meßwerte heranzuziehen. - Ebenso ist es möglich, für alle Detektorelemente gemeinsam nur eine Summierschaltung 25, nur eine Multiplizierschaltung 26 und nur eine Subtrahierschaltung 27 zu verwenden, wenn die den Detektorelementen zugeordneten Zwischenspeicher im Multiplexbetrieb daran angeschlossen werden. In diesem Fall liefert die Subtrahierschaltung 27 sequentiell die korrigierten Meßwerte.

Die Rekonstruktion der Dichteverteilung 28 in der Rechen- und Speichereinrichtung anhand der auf diese Weise korrigierten Meßwerte ist relativ einfach, weil jeder Meßwert im Prinzip schon die Dichte in einem bestimmten Punkt darstellt, wie schon in der DE-OS 27 13 581 ausgeführt. An die Rechen- und Speichereinrichtung 28 ist eine Wiedergabeeinrichtung 29, z.B. mit einer Kathodenstrahlröhre, angeschlossen, die die Dichteverteilung optisch darstellt.

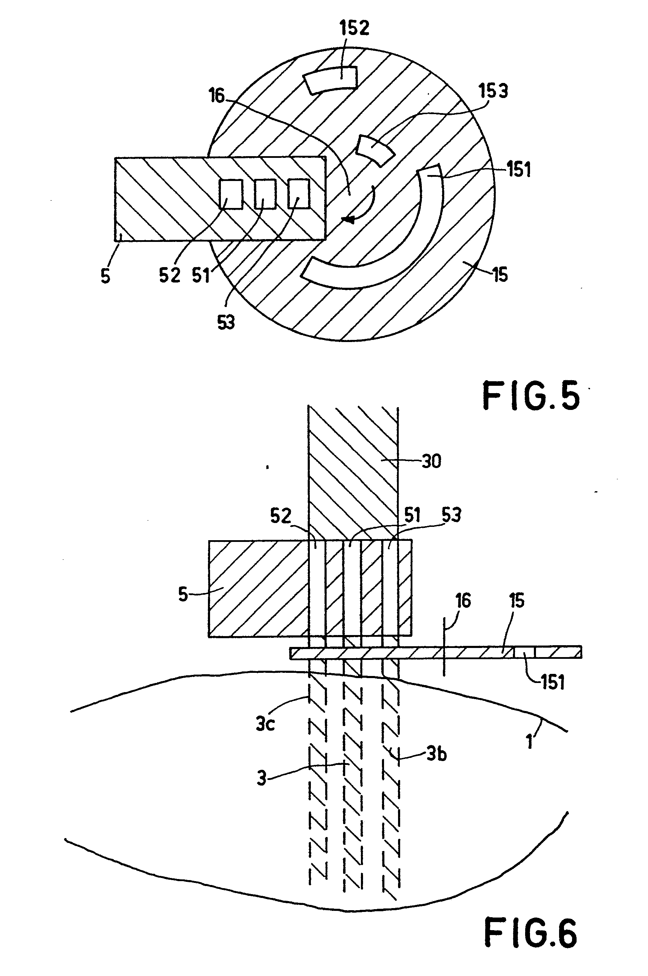

Die zur Erfassung der Mehrfach-Streustrahlung erforderliche Relativverschiebung zwischen Primärstrahl einerseits und Blendenanordnung mitsamt Detektoranordnung und Lamellen andererseits kann wie erwähnt auch durch eine Verschiebung des Primärstrahles bei feststehender Blendenanordnung erreicht werden. Zu diesem Zweck muß das von den beiden Röntgenstrahlern 4a und 4b (Fig. 1) erzeugte Röntgenstrahlenbündel dem Untersuchungsbereich über je eine Einrichtung zugeführt werden, wie sie in den Fig. 5 und 6 dargestellt ist, wobei Fig. 5 diese Einrichtung in einer zum Primärstrahl senkrechten Ebene und Fig. 6 die Einrichtung in der Ebene des Primärstrahles darstellt.

Das Primärstrahlenbündel 30, das zunächst noch einen relativ großen Querschnitt aufweist (Fig. 6), fällt auf eine feststehende Kollimatorblende 5, die mit drei parallelen, auf einer Linie nebeneinanderliegenden Kanälen 51, 52 und 53 versehen ist. Das Primärstrahlenbündel 30 wird dadurch in drei Primärstrahlen 3, 3c und 3b aufgespalten (Fig. 6). Die drei auf diese Weise erzeugten Primärstrahlen treffen auf eine kreisförmige Absorberscheibe 15, die - angetrieben durch einen nicht näher dargestellten Motor - um eine zu den Primärstrahlen parallele, mit deren Mittelpunkten annähernd in einer Ebene liegende Achse 16 rotiert. Die Absorberscheibe 15 ist mit drei kreisförmigen auf dem Umfang gegeneinander wersetzten und in verschiedenem Abstand voneinander angeordneten Ausnehmungen 151, 152 und 153 versehen, die so angeordnet sind, daß bei einer Drehung der Absorberscheibe 15 im Uhrzeigersinn zunächst die Ausnehmung 151 hinter dem Kanal 51 erscheint und den Primärstrahl 3 durchläßt, danach die Ausnehmung 153 unter dem Kanal 53, die den Primärstrahl 3b durchläßt und schließlich die Ausnehmung 152 unter dem Kanal 52, die den Primärstrahl 3c durchläßt. Geht man davon aus, daß die Blenden-, Lamellen- und Detektoranordnung, die derjenigen nach Fig. 3 entspricht, feststehend und so angeordnet ist, daß sich die Lamellenebenen im Primärstrahl 3 schneiden, dann wird zunächst also (solange die Ausnehmung 151 unter dem Kanal 51 vorbeiläuft) die gesamte Streustrahlung (Einfach- und Mehrfach-Streustrahlung) won der Detektoranordnung gemessen und anschließend - solange eine der beiden Öffnungen 153 oder 152 unter dem Kanal 53 bzw. 52 vorbeiläuftdie Mehrfach-Streustrahlung. Wenn wie bei der Anordnung nach Fig. lmit zwei Röntgenstrahlern gearbeitet wird, muß dafür Sorge getragen werden, daß die Absorberscheiben auf beiden Seiten des Untersuchungsbereiches synchron laufen, so daß sich die ausgeblendeten Primärstrahlen jeweils räumlich und zeitlich decken.

Wie aus Fig. 5 ersichtlich, erstreckt sich die Ausnehmung 151 über einen wesentlich größeren Umfangswinkel als die Ausnehmungen 152 und 153, so daß - konstante Rotationsgeschwindigkeit vorausgesetzt - der Primärstrahl 3 das Objekt 1 entsprechend dem Verhältnis der Umfangswinkel zwischen der Öffnung 151 und den beiden anderen Öffnungen länger durchstrahlt als die Primärstrahlen 3c und 3b. Dadurch können bei der Erfassung der Gesamt-Streustrahlung mehr Photonen erfaßt werden, was die Genauigkeit erhöht.

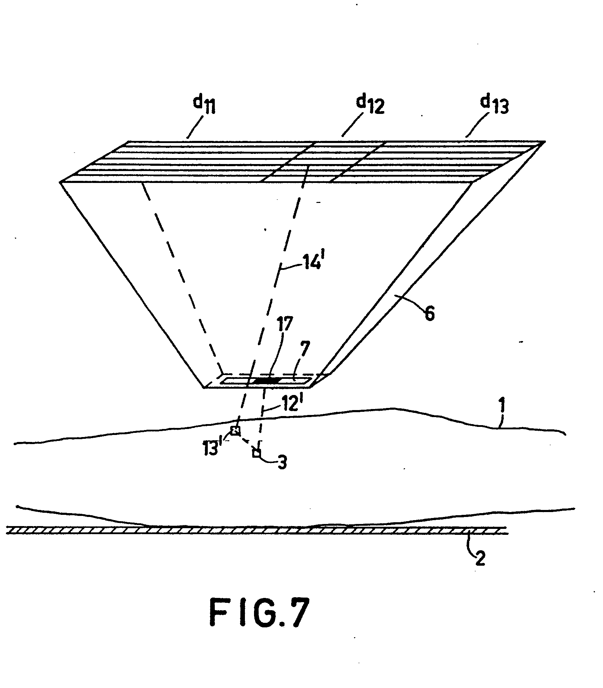

In Fig. 7 ist eine Ausführungsform dargestellt, bei der Lamellen zur Ausblendung eines Großteils der Mehrfach-Streustrahlung nicht erforderlich sind. Die Blendenanordnung 6 ist dabei aber genauso ausgebildet wie bei der Anordnung nach Fig. 1, d.h. sie ist mit einer schlitzförmigen Öffnung 7 wersehen, deren Längsrichtung senkrecht zum Primärstrahl 3 verläuft. Die Detektoranordnung besteht auch hier aus streifenförmigen Einzelelementen, jedoch sind diese in Längsrichtung in drei Teile (das Element $d_1$ der Fig. 1 ist z.B. in die drei Teile $d_{11}$, $d_{12}$ und $d_{13}$) geteilt, deren Ausgangssignale getrennt voneinander verarbeitet werden. In der schlitzförmigen Öffnung 7 ist ein Absorberstück 17 angeordnet, das einen Teil, worzugsweise den mittleren ($d_{12}$ und die ihm entsprechenden Elemente der anderen Detektorstreifen), gegen Streustrahlung, die im Primärstrahl erzeugt wird, abschirmt, während die im Primärstrahl erzeugte Streustrahlung die beiden anderen Teile $d_{11}$, $d_{13}$

und die ihnen entsprechenden Elemente der anderen Detektorstreifen direkt treffen kann. Die beiden letztgenannten Teile messen daher die Gesamt-Streustrahlung (Einfach- + Mehrfach-Streustrahlung), während der mittlere Teil nur die Mehrfach-Streustrahlung mißt, die z.B. im Punkt 13' erzeugt wird und entsprechend den Strahlen 14' an dem Absorberstück 17 vorbei durch den Schlitz 14' den mittleren Teil der Detektoranordnung ($d_{12}$ usw.) trifft.

Die von den Elementen $d_{11}$ und $d_{13}$ ermittelten Meßwerte können summiert werden, wonach von dem Summenwert der Meßwert des mittleren Detektorelementes $d_{12}$ - mit geeigneter Gewichtung entsprechend den wirksamen Meßflächen - subtrahiert wird.

Die Ausführungsform nach Fig. 7 hat gegenüber den vorher beschriebenen Ausführungsformen den Vorteil, daß eine mechanische Bewegung nicht erforderlich ist. Dafür ist eine höhere Zahl von Detektorelementen erforderlich.

Bisher wurde die Erfindung in Verbindung mit Anordnungen beschrieben, wie sie aus der DE-OS 27 13 581 und 27 57 320 bekannt sind. Die Erfindung ist jedoch auch bei anderen Anordnungen z.B. gemäß der DE-OS 26 55 230 anwendbar.

## Patentansprüche

1. Verfahren zur Korrektur der mit Hilfe einer aus mehreren Detektorelementen bestehenden Detektoranordnung bei der Durchstrahlung eines Körpers mit durchdringender Strahlung gemessenen Werte, bei dem ein Primärstrahl mit im Vergleich zu diesem geringem Querschnitt den Körper durchsetzt und die dabei erzeugte Streustrahlung durch eine Blendenanordnung ausgeblendet und durch die Detektoranordnung gemessen wird, wonach aus den Meßwerten die Dichteverteilung im vom Primärstrahl durchsetzten Bereich des Körpers rekonstruiert wird, dadurch gekennzeichnet, daß wenigstens ein Teil der Elemente der Detektoranordnung zumindest zeitweise gegen die Einfach- Streustrahlung abgeschirmt wird und daß die dabei von den abgeschirmten Elementen gemessenen Werte der Mehrfach-Streustrahlung zur Korrektur der bei der Messung der Einfach-Streustrahlung erzeugten Meßwerte herangezogen werden.

2. Anordnung zur Durchführung des Verfahrens nach Anspruch 1 mit wenigstens einer Strahlenquelle (4a, 4b) zur Erzeugung des Primärstrahles (3), mit einer Detektoranordnung (D) zur Messung der im Primärstrahl erzeugten Streustrahlung mit einer zwischen der Detektoranordnung und dem Primärstrahl angeordneten Blendenanordnung (6) mit schlitzförmiger mit ihrer Längsrichtung senkrecht zum Primärstrahl verlaufender Blendenöffnung (7), und mit einer Vielzahl zwischen der Blendenöffnung und der Detektoranordnung angeordneter Lamellen (20) aus die Strahlung absorbierendem Material, die in sich im Körper (3) schneidenden Ebenen liegen, gekennzeichnet durch Mittel (18; 5, 15) zur Erzeugung einer Relativbewegung zwischen dem Primärstrahl (3) einerseits und der Blendenanordnung (6) der Detektoranordnung (D) und den Lamellen (20) andererseits derart, daß die Lawellenebenen von einer Position, in der sie sich im Primärstrehl schneiden, in eine Position bewegbar sind, in der sie sich außerhalb des Primärstrahls (3) schneiden.

3. Anordnung nach Anspruch 2, gekennzeichnet durch eine Antriebseinrichtung (18) zur Verschiebung der Detektoranordnung (D) mitsamt der Blendenanordnung (6) und den Lamellen (20) in Längsrichtung der schlitzförmigen Blendenöffnungen (7). (Fig. 3)

4. Anordnung nach Anspruch 2, dadurch gekennzeichnet, daß vor der Strahlenquelle eine feststehende Blende (5) angeordnet ist mit wenigstens zwei Öffnungen (51...53) zum Ausblenden des Primärstrahles, daß vor dieser Blende eine mit einer Antriebseinrichtung verbundene drehbare Absorberscheibe (15) angeordnet ist, die mit bezüglich der Drehachse (16) kreisbogenförmigen Ausnehmungen (151...153) versehen ist, die gegeneinander auf dem Umfang versetzt und in solchem Abstand von der Drehachse (16) angeordnet sind, daß jeweils höchstens einer der durch die Blende ausgebendeten Strahlen (3, 3b, 3c) durch eine Ausnehmung in der Absorberscheibe treten kann. (Fig. 5, 6)

5. Anordnung nach Anspruch 4, dadurch gekennzeichnet, daß die Ausnehmung (151), durch die derjenige Primärstrahl (3) hindurchtritt, in dem sich die Lamellenebenen schneiden, eine größere Bogenlänge hat als die andere(n) Ausnehmung(en) (152, 153).

6. Anordnung zur Durchführung des Verfahrens nach Anspruch 1 mit wenigstens einer Strahlenquelle zur Erzeugung des Primärstrahls, mit einer Detektoranordnung zur Messung der in Primärstrahl erzeugten Streustrahlung, mit einer zwischen der Detektoranordnung und dem Primärstrahl angeordneten Blendenanordnung mit schlitzförmiger mit ihrer Längsrichtung senkrecht zum Primärstrahl verlaufender Blendenöffnung, dadurch gekennzeichnet, daß in die Blendenöffnung enthaltenden Ebenen jeweils wenigstens zwei Detektorelemente ($d_{11}...d_{13}$) zur Erzeugung zweier getrennter Signale vorgesehen sind, von denen eines ($d_{12}$) durch ein Absorberstück (17) gegen Einfachstreustrahlung abgeschirmt ist.

7. Anordnung nach Anspruch 6, dadurch gekennzeichnet, daß das Absorberstück (17) aus dem Strahlengang entfernbar angeordnet ist.

## Claims

1. A method of correcting the values measured by means of a detector array comprising a plurality of detector elements during radiation of a body using penetrating radiation, in which a primary beam having a small cross-section in comparison with the body is directed through the body and the scattered radiation thus generated is limited by a diaphragm arrangement and is measured by the detector array, after which the density distribution in the section of the body radiated by the primary beam is reconstructed from the measured values, characterized in that at least some of the elements of the detector array are screened at least occasionally from the single scattered radiation, the values of the multiple scattered radiation then measured by the screened detector elements being used to correct measured values generated during the measurement of the single scattered radiation.

2. A device for implementing the method claimed in Claim 1, comprising at least one radiation source (4a, 4b) to generate the primary beam (3), a detector array (D) for measuring the scatttered radiation generated in the primary beam, a diaphragm arrangement (6) placed between the detector array and the primary beam, having a slitshaped aperture (7) whose longitudinal direction extends at right angles to the primary beam, and a large plurality of lamellae (20) made of radiation-absorbing material which are arranged between the diaphragm aperture and the detector array and which lie in planes which intersect in the body (3) characterized in that there are provided means (18, 5, 15) for generating a relative displacement between the primary beam (3) on the ons hand and the diaphragm arrangement (8), the detector array (D) and the lamellae (20) on the other hand, in such a way that the planes of the lamellae can be displaced from a position in which they intersect in the primary beam, to a position in which they intersect outside the primary beam (3).

3. A device as claimed in Claim 2, characterized in that there is provided a drive mechanism (18) to shift the detector array (D), together with the diaphragm arrangement (6) and the lamellae (20), in the longitudinal direction of the slit-shaped diaphragm apertures (7). (Fig. 3).

4. A device as claimed in Claim 2, characterized in that a stationary diaphragm (5) is arranged in front of the radiation source, with at least two apertures (51... 53) for forming the primary beam, a rotatable absorber disc (15) which is connected to a drive mechanism being arranged in front of this diaphragm, said disc being provided with cut-outs (151... 153) which are arc-shaped with respect to the axis of rotation and which are arranged at the circumference in staggered formation at such a distance from the axis of rotation (16) that at any instant no more than one of the beams (3, 3b, 3c) formed by the diaphragm can pass through a cut-out in the absorber disc (Figs. 5, 8).

5. A device as claimed in Claim 4, characterized in that the cut-out (151) through which that primary beam (3) passes in which the planes of the lamellae intersect, has a greater length of arc than the other cut-out or cut-outs (151, 153).

6. A device for implementing the method claimed in Claim 1, comprising at least one radiation source for the generation of the primary beam, a detector array for the measurement of the scattered radiation generated in the primary beam, a diaphragm arrangement placed between the detector array and the primary beam, having a slit-shaped aperture whose longitudinal direction extends at right angles to the primary beam, characterized in that each time at least two detector elements ($d_{11}...$ $d_{13}$) are situated in planes containing the diaphragm aperture in order to generate two separate signals, one of said detector elements ($d_{12}$) being screened by an absorber element (17) from single scattered radiation.

7. A device as claimed in Claim 6, characterized in that the absorber element (17) is arranged so as to be removable from the beam path.

## Revendications

1.- Procédé de correction des valeurs mesurées à l'aide d'un dispositif formant détecteur constitué de plusieurs éléments détecteurs lors du passage d'un rayonnement pénétrant en transmission à travers un corps, selon lequel un pinceau de rayons primaire de section faible par rapport au corps traverse le corps et le rayonnement dispersé qui est alors produit est diaphragmé par un dispositif formant diaphragme et est mesuré par le dispositif formant détecteur, apès quoi la répartition des densités dans la zòne du corps traversée par le pinceau de rayons primaire est reconstruite à partir des valeurs de mesure, caractérisé en ce qu'au moins une partie des éléments du dispositif formant détecteur est protégée au moins par moments du rayonnement de dispersion simple et que les valeurs du rayonnement de dispersion multiple mesurées dans ce cas par les éléments protégés sont utilisées pour corriger les valeurs de mesure produites lors de la mesure du rayonnement de dispersion simple.

2.- Dispositif pour réaliser le procédé suivant la revendication 1, comportant au moins une source de rayons (4a, 4b) pour produire le pinceau de rayons primaire (3), un dispositif formant détecteur (D) pour mesurer le rayonnement dispersé produit dans le pinceau de rayons primaire, un dispositif formant diaphragme (6) installé entre le dispositif formant détecteur et le pinceau de rayons primaire et présentant une ouverture de diaphragme (7) en forme de fente dont la direction longitudinale s'étend perpendiculairement au pinceau de rayons primaire, et plusieurs lamelles (20) en matière absorbant le rayonnement disposées entre l'ouverture de diaphragme et le dispositif formant détecteur, ces lamelles se situant dans des plans qui se coupent dans le corps (3), caractérisé par des moyens (18; 5, 15) pour produire un déplacement relatif entre le pinceau de rayons primaire (3) d'une part et le dispositif formant diaphragme (6), le dispositif formant détecteur et les lamelles (20) d'autre part d'une manière telle que les plans des lamelles peuvent être déplacés depuis une position dans laquelle ils se coupent dans le pinceau de rayons primaire vers une position dans laquelle ils se coupent en dehors du pinceau de rayons primaire (3).

3.- Dispositif suivant la revendication 2, caractérisé par un dispositif d'entraînement (18) servant à déplacer le dispositif formant détecteur (D) en compagnie du dispositif formant diaphragme (6) et des lamelles (20) dans le sens longitudinal des ouvertures de diaphragme en forme de fente (7) (Fig. 3).

4.- Dispositif suivant la revendication 2, caractérisé en ce que devant la source de rayons est monté un diaphragme fixe (5) présentant au moins deux ouvertures (51...53) pour diaphragmer le faisceau de rayons primaire, que devant ce diaphragme est prévu un disque absorbant rotatif (15) relié à un dispositif d'entraînement, qui est pourvu de fenêtres en arc de cercle (151...153) centrées sur l'axe de rotation (16), décalées les unes des autres sur la périphérie et situées à une distance de l'axe de rotation (16) telle qu'au maximum un des pinceaux de rayons (3, 3b, 3c) diaphragmés par le diaphragme peut chaque fois traverser une fenêtre du disque absorbant (Fig. 5, 6).

5.- Dispositif suivant la revendication 4, caractérisé en ce que la fenêtre (151) traversée par le pinceau de rayons primaire (3) dans lequel les plans des lamelles se coupent présente une longueur en arc de cercle supérieure à celle de la ou des autres fenêtres (152, 153).

6.- Dispositif pour exécuter le procédé suivant la revendication 1, comportant au moins une source de rayons pour produire le pinceau de rayons primaire, un dispositif formant détecteur pour mesurer le rayonnement dispersé produit dans le pinceau de rayons primaire, un dispositif formant diaphragme installé entre le dispositif formant détecteur et le pinceau de rayons primaire et présentant une ouverture de diaphragme en forme de fente dont le sens longitudinal s'étend perpendiculairement au pinceau de rayons primaire, caractérisé en ce que dans les plans contenant l'ouverture de diaphragme sont prévus chaque fois au moins deux éléments détecteurs ($d_{11}$...$d_{13}$) pour fournir deux signaux séparés, l'un de ces éléments ($d_{12}$) étant protégé par une pièce en matière absorbante (17) contre le rayonnement de dispersion simple.

7.- Dispositif suivant la revendication 6, caractérisé en ce que la pièce en matière absorbante (17) est prévue telle qu'elle peut être écartée du trajet des rayons.

$d_1$  $d_2$  $d_3$  $D$

7  6

4a  3  1  4b

2

## FIG.1

20  D

6

1  3

2

4a

## FIG.2

1

FIG.3

FIG.4

152

16

153

151

5

52 51

53

15

## FIG.5

52

51 53

30

5

16

15

151

3c

3

3b

1

## FIG.6

FIG.7